(19) Europäisches Patentamt
European Patent Office
Office européen des brevets

(11) **EP 2 098 261 B1**

(12) **EUROPÄISCHE PATENTSCHRIFT**

(45) Veröffentlichungstag und Bekanntmachung des Hinweises auf die Patenterteilung:
**04.12.2019 Patentblatt 2019/49**

(51) Int Cl.:
*A61N 5/06* *(2006.01)*     *A61N 5/073* *(2006.01)*
*A61M 21/00* *(2006.01)*

(21) Anmeldenummer: **08007639.1**

(22) Anmeldetag: **18.04.2008**

(54) **Vorrichtung zur visuellen Stimulation**

Device for visual stimulation

Dispositif de stimulation visuelle

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **05.03.2008 DE 102008012669**

(43) Veröffentlichungstag der Anmeldung:
**09.09.2009 Patentblatt 2009/37**

(73) Patentinhaber: **Forschungszentrum Jülich GmbH 52425 Jülich (DE)**

(72) Erfinder:
• **Tass, Peter**
  **81541 München (DE)**
• **Barnikol, Birgit Utako**
  **52445 Titz (DE)**
• **Hauptmann, Christian**
  **52224 Stolberg (DE)**
• **Haroud, Karim**
  **1512 Chavannes-sur-Moudon/VD (CH)**
• **Roulet, Jean-Christophe**
  **2523 Ligniéres/NE (CH)**
• **Schnell, Urban**
  **3053 Münchenbuchsee/BE (CH)**

(74) Vertreter: **Müller-Boré & Partner Patentanwälte PartG mbB Friedenheimer Brücke 21 80639 München (DE)**

(56) Entgegenhaltungen:
WO-A2-01/64005     DE-A1- 10 110 004
DE-A1- 10 233 960   DE-A1- 19 543 405
DE-A1- 19 905 145   US-A- 5 709 645
US-A1- 2003 181 961

**Beschreibung**

Die Erfindung betrifft eine Vorrichtung zur visuellen Stimulation.

Bei zahlreichen neurologischen und psychiatrischen Erkrankungen kommt es im Gehirn zu übermäßig starken Synchronisationsvorgängen neuronaler Aktivität, welche die Gehirnfunktion massiv beeinträchtigt. Derzeit verfügbare Therapieverfahren für derartige Krankheiten sind z.B. die Pharmatherapie und die tiefe Hirnstimulation.

Die Druckschrift WO 01/64005 A2 offenbart in Fig. 2 und der zugehörigen Beschreibung auf Seite 2, vierter Absatz sowie Seite 7, dritter und vierter Absatz eine Vorrichtung, die eine Steuereinheit und eine Stimulationseinheit mit einer Mehrzahl von Stimulationselementen, die visuelle Stimulationssignale erzeugen, umfasst. Mindestens zwei der Stimulationselemente erzeugen die visuellen Stimulationssignale an unterschiedlichen räumlichen Orten und sind an vorgegebenen Orten im Gesichtsfeld des Patienten angeordnet. Weitere Vorrichtungen zur visuellen Stimulation sind auch aus den folgenden Druckschriften bekannt: DE 195 43 405 A1, DE 199 05 145 A1, DE 101 10 004 A1.

Vor diesem Hintergrund wird eine Vorrichtung gemäß Anspruch 1 angegeben. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1    eine schematische Darstellung einer Vorrichtung 100 gemäß einem Ausführungsbeispiel;

Fig. 2    eine schematische Darstellung des Gesichtsfelds eines Patienten;

Fig. 3    eine schematische Darstellung einer Vorrichtung 300 gemäß einem weiteren Ausführungsbeispiel;

Fig. 4    eine schematische Darstellung einer Vorrichtung 400 gemäß einem weiteren Ausführungsbeispiel;

Fig. 5    eine schematische Darstellung einer Vorrichtung 500 gemäß einem weiteren Ausführungsbeispiel;

Fig. 6    eine schematische Darstellung einer Vorrichtung 600 gemäß einem weiteren Ausführungsbeispiel;

Fig. 7    eine schematische Darstellung einer Vorrichtung 700 gemäß einem weiteren Ausführungsbeispiel;

Fig. 8    eine schematische Darstellung einer Vorrichtung 800 gemäß einem weiteren Ausführungsbeispiel;

Fig. 9    eine schematische Darstellung einer Transmissionsbrille 900 mit homogenen Transmissionsgläsern;

Fig. 10    eine schematische Darstellung einer Transmissionsbrille 1000 mit segmentierten Transmissionsgläsern;

Fig. 11    eine schematische Darstellung von mittels einer Mehrzahl von Stimulationselementen erzeugten visuellen Stimulationssignalen;

Fig. 12    eine schematische Darstellung von mittels einer Mehrzahl von Stimulationselementen erzeugten visuellen Stimulationssignalen;

Fig. 13    eine schematische Darstellung von mittels einer Mehrzahl von Stimulationselementen erzeugten visuellen Stimulationssignalen;

Fig. 14    eine schematische Darstellung von mittels einer Mehrzahl von Stimulationselementen erzeugten visuellen Stimulationssignalen;

Fig. 15    eine schematische Darstellung einer partiell durchsichtigen Lichtbrille 1500;

Fig. 16    eine schematische Darstellung einer undurchsichtigen Lichtbrille 1600;

Fig. 17    eine schematische Darstellung von mittels einer Mehrzahl von Stimulationselementen erzeugten visuellen Stimulationssignalen;

Fig. 18    eine schematische Darstellung einer segmentierten Transmissionsbrille mit einer Ansteuerungselektronik;

Fig. 19    eine schematische Darstellung eines Querschnitts durch ein LCD-Transmissionsglas;

Fig. 20    eine schematische Darstellung einer segmentierten elektrisch leitfähigen Schicht;

Fig. 21    eine schematische Darstellung der Ansteuerung und des Transmissionsverhaltens eines Segments einer Transmissionsbrille;

Fig. 22    eine Darstellung eines Transmissionsspektrums einer Transmissionsbrille; und

Fig. 23    eine schematische Darstellung einer Vorrichtung 2300 gemäß einem weiteren Ausführungsbeispiel.

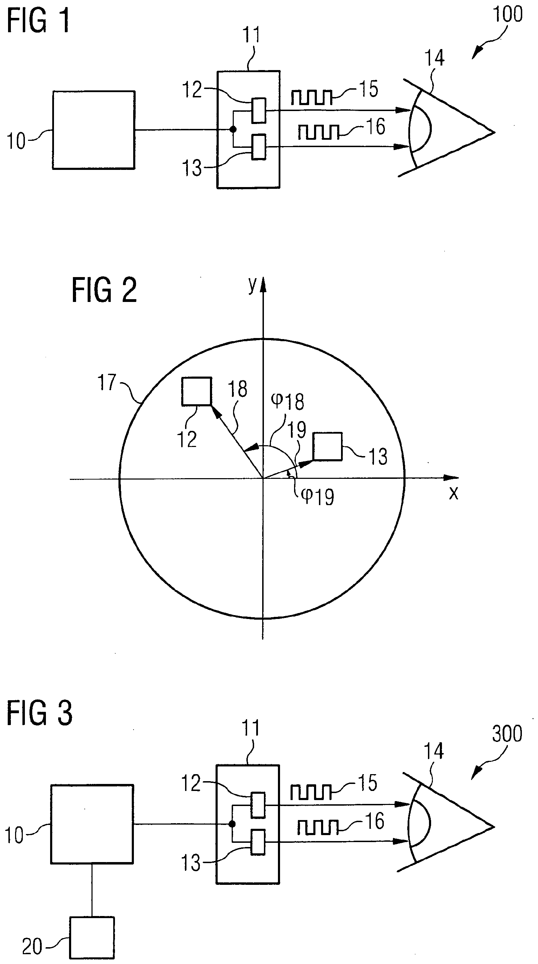

In Fig. 1 ist schematisch eine Vorrichtung 100 dargestellt, die aus einer Steuereinheit 10 und einer mit der Steuereinheit 10 verbundenen Stimulationseinheit 11 besteht. Die Stimulationseinheit 11 beinhaltet eine Mehrzahl von Stimulationselementen, die von der Steuereinheit 10 angesteuert werden. In dem vorliegenden Ausführungsbeispiel weist die Stimulationseinheit 11 zwei Stimulationselemente 12 und 13 auf. In Fig. 1 ist ferner ein Auge 14 eines Patienten dargestellt.

Während des Betriebs der Vorrichtung 100 erzeugen die Stimulationselemente 12 und 13 visuelle Stimulationssignale 15 bzw. 16, die von dem Patienten über ein oder beide Augen 14 aufgenommen werden und über die Sehnerven an Neuronenpopulationen im Gehirn weitergeleitet werden. Die Steuereinheit 10 steuert die Stimulationselemente 12 und 13 dabei derart an, dass die visuellen Stimulationssignale 15 und 16 beispielsweise zeitversetzt generiert werden.

Anstelle einer zeitversetzten Applikation der visuellen Stimulationssignale 15 und 16 können diese auch mit unterschiedlichen Phasen oder unterschiedlichen Polaritäten appliziert werden. Ferner sind auch Mischformen denkbar, d.h. die visuellen Stimulationssignale 15 und 16 können z.B. zeitversetzt sein und unterschiedliche Polaritäten aufweisen. Die Vorrichtung 100 kann so ausgestaltet sein, dass mit ihr beispielsweise nur eine der vorstehend genannten Stimulationsvarianten ausgeführt werden kann, oder die Vorrichtung 100 kann alternativ so ausgestaltet sein, dass mit ihr mehrere der genannten Stimulationsvarianten ausgeführt werden können.

Den visuellen Stimulationssignalen 15 und 16 kann eine Leuchtstärken- bzw. Helligkeitsvariation (bzw. Variation der Lichtintensität oder Lichtstärke) zugrunde liegen, beispielsweise können sie als Pulse oder als Sequenzen von Pulsen mit variierter Leuchtstärke bzw. Helligkeit appliziert werden. Die visuellen Stimulationssignale 15 und 16 können je nach Ausgestaltung der Stimulationseinheit 11 als Leuchtstärkenmodulation natürlicher visueller Reize, z.B. mittels einer homogenen oder segmentierten Transmissionsbrille, als zusätzlich zu einem natürlichen visuellen Reiz auftretender, modulierter visueller Reiz, z.B. mittels einer partiell durchsichtigen Lichtbrille, oder als künstlicher visueller Helligkeitsreiz, z.B. mittels einer undurchsichtigen Lichtbrille, verabreicht werden. Falls der Patient die visuellen Stimulationssignale über beide Augen 14 aufnimmt, können die jeweiligen Stimulationssignale beider Augen 14 korreliert bzw. koordiniert werden.

Die Vorrichtung 100 kann insbesondere zur Behandlung von neurologischen oder psychiatrischen Erkrankungen, wie z.B. Morbus Parkinson, essentiellem Tremor, Dystonie, Epilepsie, Tremor infolge von Multipler Sklerose sowie anderen pathologischen Tremores, Depression, Zwangserkrankungen, Tourette-Syndrom, Dysfunktion nach Schlaganfall, Tinnitus, Schlafstörungen, Schizophrenie, Suchterkrankungen, Persönlichkeitsstörungen, Aufmerksamkeits-Defizit-Syndrom, Aufmerksamkeits-Defizit-Hyperaktivitäts-Syndrom, Spielsucht, Neurosen, Fresssucht, Burnout-Syndrom, Fibromyalgie, Migräne, Cluster-Kopfschmerz, allgemeiner Kopfschmerz, Neuralgie, Ataxie, Tic-Störung oder Hypertonie, aber auch anderen Krankheiten verwendet werden.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d.h. die beteiligten Neuronen feuern übermäßig synchron. Hinzu kommt, dass die kranke Neuronenpopulation eine oszillatorische neuronale Aktivität aufweist, d.h. die Neuronen feuern rhythmisch. Bei den oben genannten Krankheiten liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen qualitativ anders, z.B. auf unkontrollierte Weise.

Die von den Stimulationselementen 12 und 13 erzeugten visuellen Stimulationssignale 15 und 16 sind derart ausgestaltet, dass sie, wenn sie von der Retina aufgenommen werden und über den Sehnerv zu einer Neuronenpopulation mit einer krankhaft synchronen und oszillatorischen Aktivität geleitet werden, in der Neuronenpopulation ein Zurücksetzen, einen sogenannten Reset, der Phase der neuronalen Aktivität der stimulierten Neuronen bewirken. Durch das Zurücksetzen wird die Phase der stimulierten Neuronen unabhängig von dem aktuellen Phasenwert auf einen bestimmten Phasenwert, z.B. 0°, gesetzt. Somit wird die Phase der neuronalen Aktivität der krankhaften Neuronenpopulation mittels einer gezielten Stimulation kontrolliert. Ferner ist es aufgrund der Mehrzahl von Stimulationselementen möglich, die krankhafte Neuronenpopulation an unterschiedlichen Stellen zu stimulieren. Dies ermöglicht es, die Phase der neuronalen Aktivität der krankhaften Neuronenpopulation an den unterschiedlichen Stimulationsstellen zu unterschiedlichen Zeitpunkten zurückzusetzen. Im Ergebnis wird dadurch die krankhafte Neuronenpopulation, deren Neuronen zuvor synchron und mit gleicher Frequenz und Phase aktiv waren, in mehrere Subpopulationen aufgespalten. Innerhalb einer Subpopulation sind die Neuronen weiterhin synchron und feuern auch weiterhin mit derselben pathologischen Frequenz, aber jede der Subpopulationen weist bezüglich ihrer neuronalen Aktivität die Phase auf, die ihr durch den Stimulationsreiz aufgezwungen wurde.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d.h. die komplette Desynchronisation, ist somit nach der Applikation der Stimulationssignale über die Stimulationseinheit 11 nicht sofort vorhanden, sondern stellt sich meist innerhalb weniger Perioden oder gar in weniger als einer Periode der pathologischen Aktivität ein.

Bei der vorstehend beschriebenen Art der Stimulation wird die letztlich gewünschte Desynchronisation durch die krankhaft gesteigerte Interaktion zwischen den Neuronen erst ermöglicht. Hierbei wird ein Selbstorganisationsprozess ausgenutzt, der für die krankhafte Synchronisation verantwortlich ist. Derselbe bewirkt, dass auf eine Aufteilung einer Gesamtpopulation in Subpopulationen mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die Stimulation mit der Vorrichtung 100 eine Neuorganisation der Konnektivität der gestörten neuronalen Netzwerke erzielt werden, sodass lang anhaltende therapeutische Effekte bewirkt werden können.

In Fig. 2 ist schematisch das Gesichtsfeld 17 eines Patienten dargestellt. Als Gesichtsfeld wird der Raum bezeichnet, der mit einem Auge ohne Augenbewegungen überblickt wird. In Fig. 2 ist das Gesichtsfeld 17 zur Vereinfachung kreisförmig dargestellt. Typischerweise hat das Gesichtsfeld eine eher gewölbte ovale Form. Die genaue Größe und Form des Gesichtsfelds unterliegt dabei individuellen Schwankungen und ist zudem altersabhängig.

Punkte im Gesichtsfeld 17 lassen sich beispielsweise mit Hilfe ihrer Polarkoordinaten beschreiben. In Fig. 2 sind die räumlichen Lagen der Stimulationselemente 12 und 13 im Gesichtsfeld 17 beispielhaft dargestellt. Zur Veranschaulichung ist jeweils ein Eckpunkt der Stimulationselemente 12 und 13 mit einem Vektor 18 bzw. 19 gekennzeichnet. Die Vektoren 18 und 19 lassen sich im Polarkoordinatensystem über ihren Betrag und den Winkel $\varphi_{18}$ bzw. $\varphi_{19}$, den sie mit der x-Achse einschließen beschreiben.

Unterschiedliche Stellen im Gesichtsfeld 17 werden über die Linse des Auges auf unterschiedliche Stellen der Retina abgebildet. Die unterschiedlichen Stellen der Retina sind wiederum über den Sehnerv mit unterschiedlichen Neuronen im Gehirn verbunden. Dies bedeutet, dass mit den an unterschiedlichen räumlichen Orten angeordneten Stimulationselementen 12 und 13 jeweils unterschiedliche Neuronen stimuliert werden können. Folglich können die Stimulationselemente 12 und 13 sowie evtl. weitere Stimulationselemente räumlich so im Gesichtsfeld 17 des Patienten angeordnet sein, dass die von der Retina aufgenommenen Stimulationssignale an unterschiedliche Zielgebiete im Gehirn weitergeleitet werden. Es können demnach unterschiedliche Subpopulationen einer krankhaften Neuronenpopulation mit den Stimulationselementen 12 und 13 gezielt stimuliert werden, und es kann ein zeitversetztes Zurücksetzen der Phasen dieser Subpopulation durchgeführt werden.

Die Zuordnung der Bereiche des Gesichtsfelds zu entsprechenden Bereichen des Gehirns ist beispielsweise in dem Artikel "Visual Field Maps in Human Cortex" von B. A. Wandell, S. O. Dumoulin und A. A. Brewer, erschienen in Neuron 56, Oktober 2007, Seiten 366 bis 383, beschrieben.

Die Vorrichtung 100 kann beispielsweise in einem sogenannten "open loop"-Modus betrieben werden, bei welchem die Steuereinheit 10 die Stimulationseinheit 11 derart ansteuert, dass die Stimulationselemente 12 und 13 vorgegebene visuelle Stimulationssignale 15 und 16 erzeugen. Des Weiteren kann die Vorrichtung 100 auch zu einer in Fig. 3 gezeigten Vorrichtung 300 weitergebildet werden, welche ein sogenanntes "closed loop"-System darstellt. Die Vorrichtung 300 enthält zusätzlich zu den aus Fig. 1 bekannten Komponenten noch eine Messeinheit 20, welche am Patienten aufgenommene Messsignale bereitstellt und diese an die Steuereinheit 10 weiterleitet. Es kann vorgesehen sein, dass die Steuereinheit 10 anhand der von der Messeinheit 20 aufgenommenen Messsignale die Stimulationseinheit 11 ansteuert. Bei der Messeinheit 20 kann es sich um nicht-invasive Sensoren handeln, wie z.B. Elektroenzephalographie (EEG)-Elektroden, Magnetenzephalographie (MEG)-Sensoren, Akzelerometer, Elektromyographie (EMG)-Elektroden und Sensoren zur Bestimmung von Blutdruck, Atmung oder Hautleitwiderstand. Ferner kann die Messeinheit 20 in Form eines oder mehrerer Sensoren in den Körper des Patienten implantiert sein. Als invasive Sensoren können beispielsweise epikortikale, intrakortikale oder subkutane Elektroden dienen. Insbesondere kann mittels der Messeinheit 20 die physiologische Aktivität in dem stimulierten Zielgebiet oder einem damit verbundenen Gebiet gemessen werden.

Hinsichtlich des Zusammenwirkens der Steuereinheit 10 mit der Messeinheit 20 sind verschiedene Ausgestaltungen denkbar. Beispielsweise kann von der Steuereinheit 10 eine bedarfsgesteuerte Stimulation durchgeführt werden. Hierzu detektiert die Steuereinheit 10 anhand der von der Messeinheit 20 aufgenommenen Messsignale das Vorhandensein und/oder die Ausprägung eines oder mehrerer krankhafter Merkmale. Beispielsweise kann die Amplitude oder der Betrag der neuronalen Aktivität gemessen werden und mit einem vorgegebenen Schwellwert verglichen werden. Die Steuereinheit 10 kann so ausgestaltet sein, dass eine Stimulation eines oder mehrerer Zielgebiete gestartet wird, sobald der vorgegebene Schwellwert überschritten wird. Ferner können von der Steuereinheit 10 anhand der Ausprägung der krankhaften Merkmale Parameter der visuellen Stimulationssignale, wie beispielsweise die Stärke (Amplitude) der Stimulationssignale oder die Frequenz der Stimulation oder die Pausen zwischen den Stimulationssequenzen, eingestellt werden. Z.B. können ein oder mehrere Schwellwerte vorgegeben werden, und bei einem Überschreiten oder Unterschreiten der Amplitude oder des Betrags der Messsignale über bzw. unter einen bestimmten Schwellwert variiert die Steuereinheit 10 einen bestimmten Parameter der visuellen Stimulationssignale.

Des Weiteren kann vorgesehen sein, dass die von der Messeinheit 20 aufgenommenen Messsignale direkt oder gegebenenfalls nach einem oder mehreren Verarbeitungsschritten in visuelle Stimulationssignale umgesetzt werden und von der Stimulationseinheit 11 appliziert werden. Beispielsweise können die Messsignale verstärkt und gegebenenfalls nach mathematischer Verrechnung (z.B. nach Mischung der Messsignale) mit einer Zeitverzögerung und linearen und/oder nichtlinearen Verrechnungsschritten als Steuersignale in die Steuereingänge der Stimulationselemente 12 und 13 eingespeist werden. Der Verrechnungsmodus wird hierbei so gewählt, dass der krankhaften neuronalen Aktivität entgegengewirkt wird und die Stimulationssignale mit abnehmender krankhafter neuronaler Aktivität ebenfalls verschwinden oder zumindest deutlich in ihrer Stärke reduziert werden.

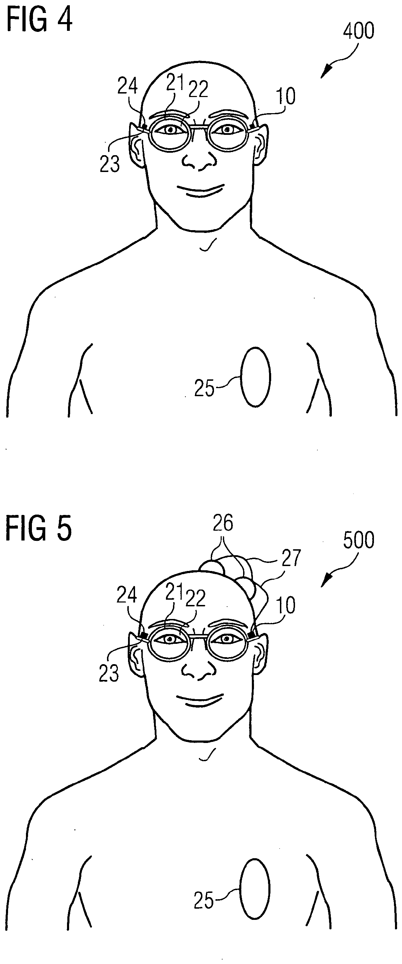

In Fig. 4 ist schematisch eine Vorrichtung 400 gezeigt, die eine Weiterbildung der in Fig. 1 gezeigten Vorrichtung 100 darstellt. Keine Komponente der Vorrichtung 400 muss implantiert werden, sodass sich die gesamte Vorrichtung 400 außerhalb des Körpers des Patienten befindet. Außerdem verwendet die Vorrichtung 400 kein von einem Sensor gemessenes Signal zur bedarfsgesteuerten Variation der Stimulation. Als Stimulationseinheit 11 wird bei der Vorrichtung 400 eine Transmissionsbrille verwendet, die aus den folgenden Komponenten besteht: (i) zwei Einfassungsteilen 21 mit je einem transmissionsmodulierten Brillenglas 22 (für jedes Auge einzeln), (ii) zwei Ohrbügeln 23, mit welchen die Brille hinter dem Ohr des Patienten mechanisch gehalten wird, und (iii) der Steuereinheit 10, welche die Transmission der transmissionsmodulierten Gläser der Brille steuert. Anstelle einer Transmissionsbrille könnte auch eine der weiter unten beschriebenen Brillen, wie z.B. eine partiell durchsichtige oder undurchsichtige Lichtbrille, als Stimulationsbrille verwendet werden. Eine Batterie oder ein Akku zur Stromversorgung der elektrischen Komponenten der Vorrichtung 400 können in der Steuereinheit 10 oder auch baulich getrennt von der Steuereinheit 10 in oder an der Brille untergebracht sein. Die Brille kann vom Patienten mittels einer Bedieneinheit 24 (z.B. Anschaltknopf und/oder Drehregler) angeschaltet werden. Mit dem Drehregler kann z.B. die maximale Stimulationsstärke eingestellt werden. Zusätzlich zu den vorstehend genannten Komponenten kann die Vorrichtung 400 über ein Steuermedium 25 verfügen, welches beispielsweise telemetrisch oder über ein Verbindungskabel mit der Steuereinheit 10 verbunden ist. Im Falle einer Verbindung über ein Kabel können Steckverbindungen zur Konnektierung bzw. Dekonnektierung verwendet werden.

Ferner kann die Vorrichtung 400 auch über ein weiteres, z.B. vom Arzt zu bedienendes Steuermedium (nicht dargestellt) verfügen, welches telemetrisch oder über ein Verbindungskabel mit der Steuereinheit 10 verbunden ist. Im Falle einer Verbindung über ein Kabel können Steckverbindungen zur Konnektierung bzw. Dekonnektierung verwendet werden.

Des Weiteren können ein oder mehrere Sensoren, z.B. EEG-Elektroden oder ein Akzelerometer, zur Registrierung und/oder Dokumentation des Stimulationserfolgs und zur Untersuchung durch den Arzt vorgesehen sein.

In den Fig. 5 bis 8 sind schematisch Vorrichtungen 500, 600, 700 und 800 als Weiterbildungen der Vorrichtung 300 dargestellt. Die Vorrichtungen 500 bis 800 verfügen jeweils über eine Messeinheit, mit der sich eine Bedarfssteuerung oder eine Rückkopplung (Feedback) der Messsignale in die Stimulationseinheit durchführen lässt. Hierbei stellen die Vorrichtungen 500 und 600 nicht-invasive Varianten dar, während die Vorrichtungen 700 und 800 zum Teil in den Körper des Patienten implantiert werden. Wie die Vorrichtung 400 umfassen die Vorrichtungen 500 bis 800 eine homogene oder segmentierte Transmissionsbrille als Stimulationseinheit. Alternativ können auch anders ausgestaltete Stimulationseinheiten verwendet werden, z.B. die weiter unten beschriebenen partiell durchsichtigen oder undurchsichtigen Lichtbrillen.

Die in Fig. 5 dargestellte Vorrichtung 500 verfügt neben den oben beschriebenen Komponenten der Vorrichtung 400 über epikutane, d.h. auf der Haut des Patienten befestigte EEG-Elektroden 26, die über Verbindungskabel 27 mit der Steuereinheit 10 verbunden sind. Die Steuereinheit 10 verstärkt die mittels der EEG-Elektroden 26 gemessene Potentialdifferenz und verwendet diese nach einer optionalen linearen oder nicht-linearen Verrechnung zur Ansteuerung der transmissionsmodulierten Gläser der Transmissionsbrille. Als Alternative zu den Verbindungskabeln 27 können die EEG-Elektroden 26 auch schnurlos, d.h. telemetrisch mit der Steuereinheit 10 verbunden sein. Dies hat den Vorteil, dass der Patient nicht durch Verbindungskabel behindert wird und z.B. an Hindernissen hängen bleiben kann.

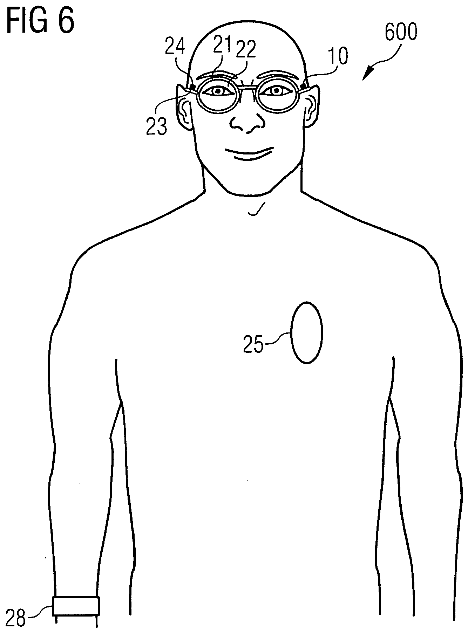

Die in Fig. 6 dargestellte Vorrichtung 600 weist anstelle einer EEG-Elektrode ein Akzelerometer (Beschleunigungsmesser) 28 als Messeinheit auf. Der Akzelerometer 28 ist an einer krankheitsbedingt zitternden Gliedmaße des Patienten, z.B. in der Art einer Uhr, befestigt. Die von dem Akzelerometer 28 aufgenommenen Beschleunigungssignale werden in der Steuereinheit 10 verstärkt und nach einer optionalen linearen oder nicht-linearen Verrechnung zur Ansteuerung der transmissionsmodulierten Gläser der Transmissionsbrille verwendet. Der Akzelerometer 28 kann telemetrisch oder über ein Verbindungskabel mit der Steuereinheit 10 verbunden sein.

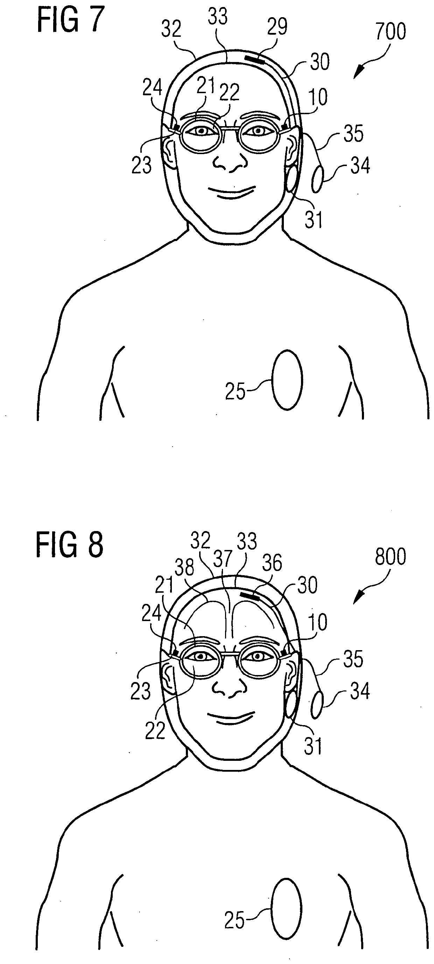

In Fig. 7 ist eine invasive Variante gezeigt. In dem dargestellten Ausführungsbeispiel umfasst die Vorrichtung 700 eine oder mehrere subkutan implantierte Elektroden 29 als Messeinheit, ein Verbindungskabel 30 und eine Sende- und Empfangseinheit 31, die im Körper des Patienten unter der Kopfhaut 32 und außerhalb des knöchernen Schädels 33 implantiert sind. Außerhalb des Körpers des Patienten befinden sich eine Sende- und Empfangseinheit 34, die über ein Verbindungskabel 35 mit der Steuereinheit 10 verbunden ist. Über die Sende- und Empfangseinheiten 31 und 34, die beispielsweise jeweils als Spule implementiert sind und zwischen denen drahtlos sowie bidirektional Signale als auch elektrische Leistung übertragen werden können, werden die von der Elektrode 29 aufgenommenen Messsignale an die Steuereinheit 10 weitergeleitet. In der Steuereinheit 10 werden die von der Elektrode 29 gemessenen Potentialdifferenzen verstärkt und nach einer optionalen linearen oder nicht-linearen Verrechnung zur Ansteuerung der transmissionsmodulierten Gläser der Transmissionsbrille herangezogen.

Ein weitere invasive Variante ist schematisch in Fig. 8 dargestellt. Bei der dort gezeigten Vorrichtung 800 dienen eine oder mehrere epikortikal implantierte Elektroden 36 als Messeinheit. "Epikortikal" bedeutet "auf der Hirnrinde gelegen"; zur Illustration ist in Fig. 8 der mit der mit dem Bezugszeichen 37 gekennzeichnete Raum zwischen der Hirnrinde 38 und der Schädeldecke 33 eingezeichnet. Die Hirnrinde 38 beider Hemisphären ist schematisch gezeigt. Die Steuereinheit 10 verstärkt die mittels der epikortikal implantierten Elektrode 36 gemessene Potentialdifferenz und verwendet diese nach einer optionalen linearen oder nicht-linearen Verrechnung zur Ansteuerung der transmissionsmodulierten Gläser der Transmissionsbrille.

Die in Fig. 8 gezeigte epikortikale Elektrode 36 kann beispielsweise auch durch eine intrakortikale Elektrode ersetzt werden (nicht dargestellt).

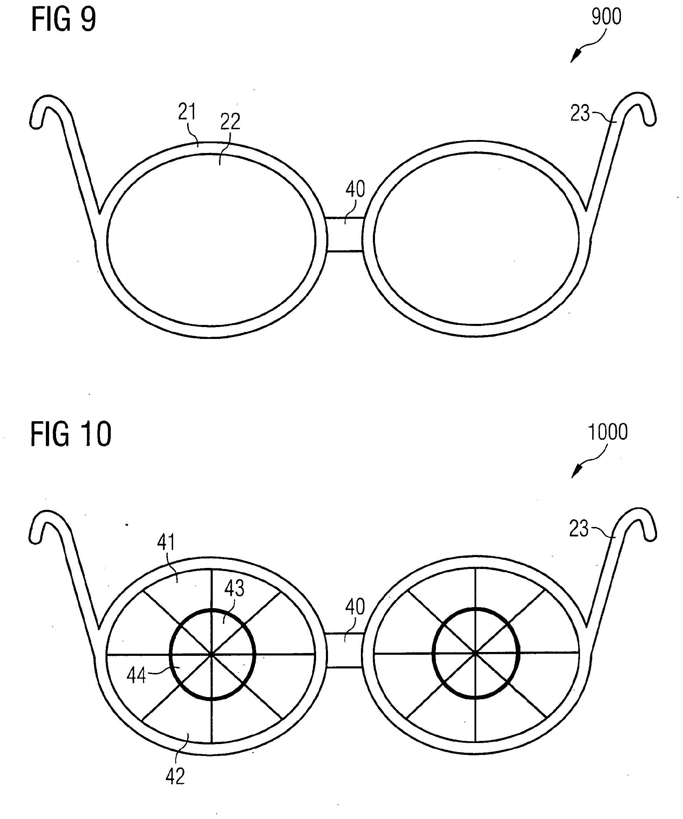

In Fig. 9 ist eine Transmissionsbrille 900 mit homogenen Transmissionsgläsern 22 dargestellt. Die Transmissionsbrille 900 umfasst ferner Ohrbügel 23 zur mechanischen Befestigung am Patientenkopf, einen Steg 40, welcher die beiden Transmissionsgläser 22 verbindet, und Einfassungsteile 21, in welche die Transmissionsgläser 22 eingefasst sind. Die Transmissionsgläser 22 sind homogen, d.h. nicht in unterschiedliche Segmente unterteilt. Die Transmission des rechten und des linken Transmissionsglases 22 können separat geregelt werden, d.h. die Transmissionsgläser 22 können als Stimulationselemente 12 und 13 im Sinne der Vorrichtung 100 verwendet werden. Mittels der Transmissionsbrille 900 können beide Augen des Patienten mit jeweils unterschiedlichen visuellen Reizen stimuliert werden.

In Fig. 10 ist eine Transmissionsbrille 1000 mit segmentierten Transmissionsgläsern 22 dargestellt. Die Transmissionsgläser 22 sind jeweils in unterschiedliche Segmente unterteilt, deren Transmission getrennt gesteuert werden kann. Die Segmentierung kann beispielsweise radial und/oder zirkular sein (beides ist in Fig. 10 gezeigt). Die in Fig. 10 gezeigte Ausführung einer segmentierten Transmissionsbrille ist lediglich beispielhaft zu verstehen. Die Anzahl der Segmente sowie die geometrischen Formen der einzelnen Segmente können anders gewählt werden.

Die Segmente der Transmissionsbrille 1000 entsprechen den in Fig. 1 gezeigten Stimulationselementen. In Fig. 10 sind vier der Segmente mit den Bezugszeichen 41, 42, 43 und 44 gekennzeichnet.

Anhand der Segmente 41 bis 44 soll nachfolgend beispielhaft erläutert werden, wie durch zeitversetztes Zurücksetzen der Phase von Subpopulationen einer krankhaft synchronen und oszillatorischen Neuronenpopulation eine Desynchronisation der gesamten Neuronenpopulation erzielt werden kann. Die Segmente 41 bis 44 sind so ausgewählt worden, dass die von ihnen erzeugten visuellen Stimulationssignale jeweils vorzugsweise von einem bestimmten Teil der Netzhaut des Patienten aufgenommen werden, von wo aus die Stimulationssignale zu bestimmten Bereichen des Gehirns weitergeleitet werden, sodass die oben beschriebene Aufspaltung einer krankhaften Neuronenpopulation in Subpopulationen ermöglicht wird. Damit Subpopulationen mit unterschiedlichen Phasen gebildet werden, können die visuellen Stimulationssignale von den Segmenten 41 bis 44 beispielsweise zeitversetzt erzeugt werden. Gleichbedeutend mit dem zeitversetzten Erzeugen der visuellen Stimulationssignale ist ein phasenversetztes Erzeugen der Stimulationssignale, welches im Ergebnis ebenfalls zu einem zeitversetzten Zurücksetzen der Phasen der unterschiedlichen Subpopulationen führt.

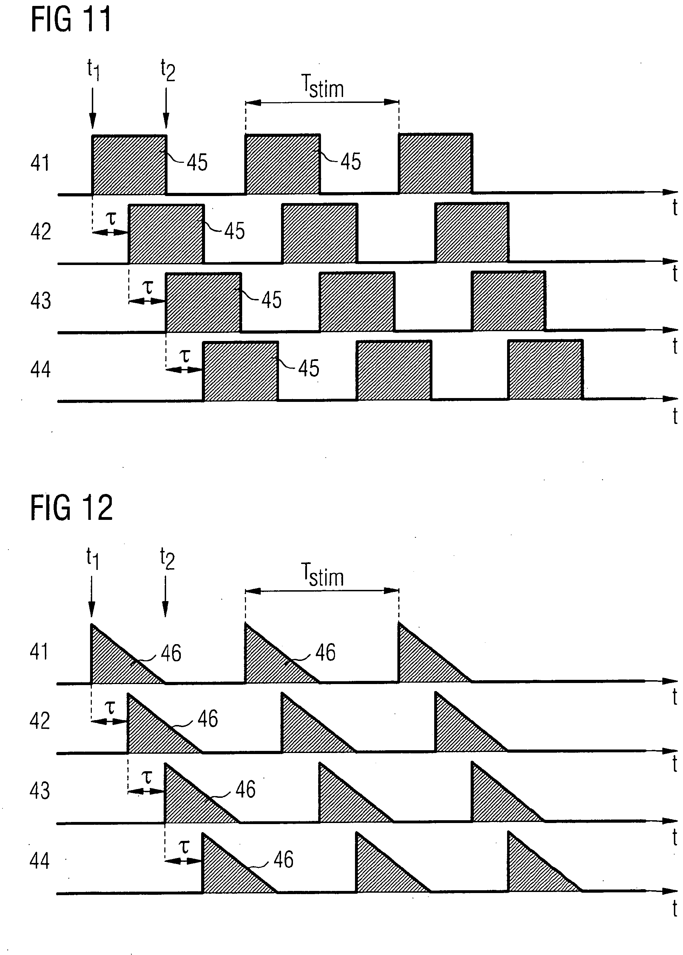

Ein für die oben beschriebenen Zwecke geeignetes Stimulationsverfahren, das beispielsweise mit einer der Vorrichtungen 100 bis 800 durchgeführt werden kann, ist in Fig. 11 schematisch dargestellt. In Fig. 11 sind untereinander die mittels der Segmente 41 bis 44 applizierten visuellen Stimulationssignale 45 gegen die Zeit t aufgetragen. Bei dem in Fig. 11 gezeigten Ausführungsbeispiel wird davon ausgegangen, dass nur die Segmente 41 bis 44 der Transmissionsbrille 1000 visuelle Stimulationssignale 45 erzeugen, d.h. nur die Transmission dieser Segmente wird von der Steuereinheit 10 moduliert. Selbstverständlich ist dies nur beispielhaft zu verstehen. Bei alternativen Ausgestaltungen können anstelle der Segmente 41 bis 44 andere Segmente zum Generieren der visuellen Stimulationssignale herangezogen werden. Es ist möglich, wie in Fig. 11 nur eine Auswahl der Segmente der Transmissionsbrille 1000 zur Stimulation zu verwenden oder auch sämtliche Segmente.

Bei dem in Fig. 11 dargestellten Verfahren appliziert jedes der Segmente 41 bis 44 periodisch das visuelle Stimulationssignal 45. Pro Segment 41 bis 44 wird das Stimulationssignal 45 in dem vorliegenden Beispiel dreimal appliziert. Alternativ könnte das Stimulationssignal 45 pro Sequenz beispielsweise auch ein- bis fünfzehnmal wiederholt

werden. Die Frequenz $f_{stim}$ = $1/T_{stim}$, mit welcher die visuellen Stimulationssignale 45 pro Segment 41 bis 44 wiederholt werden, kann im Bereich von 1 bis 30 Hz und insbesondere im Bereich von 5 bis 20 Hz liegen, kann aber auch kleinere oder größere Werte annehmen. Derartige Sequenzen von visuellen Stimulationssignalen 45 sind geeignet, die neuronale Phase einer stimulierten krankhaften Subpopulation von Neuronen zurückzusetzen.

Die Frequenz $f_{stim}$ kann beispielsweise im Bereich der mittleren Frequenz der krankhaft rhythmischen Aktivität des Ziel-Netzwerks liegen. Bei neurologischen und psychiatrischen Erkrankungen liegt die mittlere Frequenz typischerweise im Bereich von 1 bis 30 Hz, kann aber auch außerhalb dieses Bereichs liegen. Hierbei ist zu beachten, dass die Frequenz, mit welcher die krankhaften Neuronen synchron feuern, üblicherweise nicht konstant ist, sondern durchaus Variationen aufweisen kann und darüber hinaus bei jedem Patienten individuelle Abweichungen zeigt.

Zur Ermittlung der Frequenz $f_{stim}$ kann beispielsweise die mittlere Peakfrequenz der krankhaften rhythmischen Aktivität des Patienten bestimmt werden. Diese Peakfrequenz kann dann als Stimulationsfrequenz $f_{stim}$ verwendet werden oder auch variiert werden, beispielsweise in einem Bereich von $f_{stim}$ - 3 Hz bis $f_{stim}$ + 3 Hz. Alternativ kann aber auch ohne vorherige Messung eine Frequenz $f_{stim}$ im Bereich von 1 bis 30 Hz gewählt werden und diese beispielsweise während der Stimulation variiert werden, bis die Frequenz $f_{stim}$ gefunden wird, mit der sich die besten Stimulationserfolge erzielen lassen. Als weitere Alternative kann für die Stimulationsfrequenz $f_{stim}$ ein für die jeweilige Krankheit bekannter Literaturwert herangezogen werden. Eventuell kann dieser Wert noch variiert werden, bis beispielsweise optimale Stimulationsergebnisse erzielt werden.

Die Struktur eines einzelnen visuellen Stimulationssignals 45 soll nachfolgend anhand des ersten von dem Segment 41 generierten Stimulationssignals 45 erläutert werden. Hier wird zum Zeitpunkt $t_1$ das Segment 41 von der Steuereinheit 10 derart angesteuert, dass die Transmission, d.h. die Lichtdurchlässigkeit des Segments 41 minimal wird. Zum Zeitpunkt $t_2$ schaltet die Steuereinheit 10 die Transmission des Segments 41 auf den maximalen Wert. In anderen Worten bedeutet dies, dass das Segment 41 weniger transparent wird, wenn stimuliert wird. Dementsprechend nimmt der Patient eine verringerte Helligkeit des Umgebungslichts im Bereich des Segments 41 während der Stimulation wahr.

Alternativ ist es auch möglich, die Transmission des Segments 41 zum Zeitpunkt $t_1$ maximal zu schalten und zum Zeitpunkt $t_2$ minimal, sodass das Segment 41 während der Stimulation stärker transparent wird.

Grundsätzlich ist es denkbar, als maximale Transmission 100% zu wählen, d.h. in diesem Fall wird das Umgebungslicht durch das jeweilige Segment überhaupt nicht abgeschwächt. Eine derart hohe Transmission lässt sich jedoch aufgrund technischer Beschränkungen häufig nicht erreichen, sodass kleinere Transmissionswerte für die maximale Transmission im Bereich von 60% bis 100% gewählt werden können. Die minimale Transmission kann einen Wert in dem Bereich von 0% bis 30% annehmen. Es können aber auch noch Stimulationserfolge mit Transmissionswerten, die außerhalb der angegebenen Bereiche liegen, erzielt werden.

Die Dauer eines Stimulationssignals 45, d.h. die Zeitspanne zwischen den Zeitpunkten $t_1$ und $t_2$, kann beispielsweise $T_{stim}/2$ betragen. In diesem Fall sind die Zeitspanne, während der stimuliert wird, und die nachfolgende Stimulationspause gleich lang. Es ist aber auch möglich andere Stimulationsdauern zu wählen, beispielsweise im Bereich von $T_{stim}/2$ - $T_{stim}/10$ bis $T_{stim}/2$ + $T_{stim}/10$. Auch andere Stimulationsdauern sind möglich und können beispielsweise experimentell bestimmt werden.

Gemäß der in Fig. 11 gezeigten Ausgestaltung erfolgt die Verabreichung der visuellen Stimulationssignale 45 über die einzelnen Segmente 41 bis 44 der Transmissionsbrille 1000 mit einer zeitlichen Verzögerung zwischen den einzelnen Segmenten 41 bis 44. Beispielsweise kann der Beginn zeitlich aufeinander folgender und von unterschiedlichen Segmenten 41 bis 44 applizierten visuellen Stimulationssignalen 45 um eine Zeit $\tau$ verschoben sein.

Im Fall von N Stimulationselementen bzw. Segmenten, die zur Stimulation eingesetzt werden, kann die zeitliche Verzögerung $\tau$ zwischen jeweils zwei aufeinander folgenden Stimulationssignalen 45 beispielsweise im Bereich eines N-tels der Periode $T_{stim}$ = $1/f_{stim}$ liegen. In dem in Fig. 11 gezeigten Ausführungsbeispiel (N = 4) beträgt die zeitliche Verzögerung $\tau$ dementsprechend $T_{stim}/4$. Von der Vorgabe, dass die zeitliche Verzögerung $\tau$ zwischen jeweils zwei aufeinander folgenden Stimulationssignalen 45 $T_{stim}/N$ beträgt, kann bis zu einem gewissen Grad abgewichen werden. Beispielsweise kann von dem Wert $T_{stim}/N$ für die zeitliche Verzögerung $\tau$ um bis zu $\pm 10\%$, $\pm 20\%$ oder $\pm 30\%$ abgewichen werden. Bei derartigen Abweichung wurden noch Stimulationserfolge erzielt, d.h. es konnte noch ein desynchronisierender Effekt beobachtet werden.

Die in Fig. 11 dargestellte Rechteckform der Einzelpulse 45 stellt eine ideale Form dar. Je nach der Güte der die Einzelpulse 45 erzeugenden Elektronik und der Transmissionsgläser 22 wird von der idealen Rechteckform abgewichen.

Anstelle von rechteckförmigen Stimulationssignalen 45 kann die Steuereinheit 10 beispielsweise auch anders ausgestaltete Stimulationssignale erzeugen, wie sie beispielhaft in den Fig. 12 bis 14 dargestellt sind. In Fig. 12 sind dreieckförmige visuelle Stimulationssignale 46 gezeigt. Zum Zeitpunkt $t_1$ wird beispielsweise auf minimale Transmission geschaltet und bis zum Zeitpunkt $t_2$ steigt die Transmission kontinuierlich auf den maximalen Wert an. Alternativ kann vorgesehen sein, dass die Transmission zu Beginn des Stimulationssignals 46 maximal ist und anschließend auf den minimalen Wert fällt.

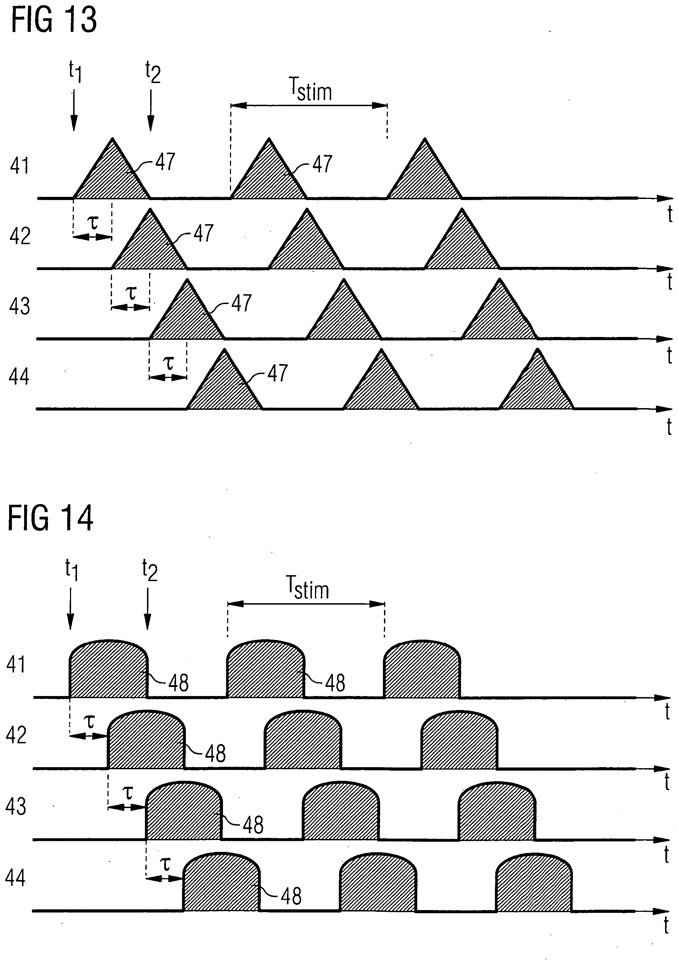

In Fig. 13 sind dreieckförmige visuelle Stimulationssignale 47 mit einer ansteigenden und einer abfallenden Flanke gezeigt. Beginnend mit dem Zeitpunkt $t_1$ wird hier die Transmission beispielsweise erhöht und nach Erreichen des Maximums bis zum Zeitpunkt $t_2$ wieder erniedrigt.

Ferner kann vorgesehen sein, dass die ansteigenden und abfallenden Flanken der visuellen Stimulationssignale (z.B. exponentiell) "abgerundet" sind. Dies ist in Fig. 14 anhand abgerundeter rechteckförmiger visueller Stimulationssignale 48 gezeigt. Darüber hinaus können die Stimulationssignale auch durch eine einfache Sinusform ersetzt werden.

Die oben beschriebenen Signalformen und deren Parameter sind nur beispielhaft zu verstehen. Es ist durchaus möglich, von den oben angegebenen Signalformen und deren Parametern abzuweichen.

Von dem in den Fig. 11 bis 14 gezeigten streng periodischen Stimulationsmuster kann auf unterschiedliche Art und Weise abgewichen werden. Beispielsweise braucht die zeitliche Verzögerung $\tau$ zwischen zwei aufeinander folgenden Stimulationssignalen 45, 46, 47 bzw. 48 nicht notwendigerweise stets gleich groß zu sein. Es kann vorgesehen sein, dass die zeitlichen Abstände zwischen den einzelnen Stimulationssignalen 45, 46, 47 bzw. 48 unterschiedlich gewählt werden. Ferner können die Verzögerungszeiten auch während der Behandlung eines Patienten variiert werden. Auch können die Verzögerungszeiten hinsichtlich der physiologischen Signallaufzeiten adjustiert werden.

Des Weiteren können während der Applikation der Stimulationssignale 45, 46, 47 bzw. 48 Pausen vorgesehen werden, während derer keine Stimulation erfolgt. Die Pausen können beliebig lang gewählt werden und insbesondere ein ganzzahliges Vielfaches der Periode $T_{stim}$ betragen. Die Pausen können nach einer beliebigen Anzahl von Stimulationen eingehalten werden. Z.B. kann eine Stimulation während N aufeinander folgender Perioden der Länge $T_{stim}$ durchgeführt werden und anschließend eine Stimulationspause während M Perioden der Länge $T_{stim}$ eingehalten werden, wobei N und M kleine ganze Zahlen sind, z.B. im Bereich von 1 bis 15. Dieses Schema kann entweder periodisch fortgesetzt werden oder stochastisch und/oder deterministisch, z.B. chaotisch, modifiziert werden.

Eine weitere Möglichkeit, von dem in den Fig. 11 bis 14 gezeigten streng periodischen Stimulationsmuster abzuweichen, besteht darin, die zeitlichen Abstände zwischen aufeinander folgenden Stimulationssignalen 45, 46, 47 bzw. 48 pro Segment 41 bis 44 stochastisch oder deterministisch oder gemischt stochastisch-deterministisch zu variieren.

Des Weiteren kann pro Periode $T_{stim}$ (oder in anderen Zeitschritten) die Reihenfolge, in welcher die Segmente 41 bis 44 die Stimulationssignale 45, 46, 47 bzw. 48 applizieren, variiert werden. Diese Variation kann stochastisch oder deterministisch oder gemischt stochastisch-deterministisch erfolgen.

Ferner kann pro Periode $T_{stim}$ (oder in einem anderen Zeitintervall) nur eine bestimmte Anzahl von den Segmenten 41 bis 44 zur Stimulation herangezogen werden und die an der Stimulation beteiligten Segmente können in jedem Zeitintervall variiert werden. Auch diese Variation kann stochastisch oder deterministisch oder gemischt stochastisch-deterministisch erfolgen.

Anstelle der in den Fig. 11 bis 14 gezeigten pulsförmigen und gegeneinander zeitverschobenen visuellen Stimulationssignale 45 bis 48 können auch visuelle Stimulationssignale mit anderen Signalformen eingesetzt werden. Beispielsweise kann jedes der Segmente 41 bis 44 ein (z.B. kontinuierliches) Sinussignal erzeugen, wobei die Phasen der von unterschiedlichen Segmenten 41 bis 44 erzeugten Sinussignale gegeneinander verschoben sind. Die mittlere Frequenz der Sinussignale kann dabei gleich sein. Die Phasenverschiebungen zwischen den einzelnen Sinussignalen können entweder vorgegeben sein, z.B. kann die Phasenverschiebung zwischen jeweils zwei von N Stimulationssignalen $T_{stim}/N$ betragen, oder die Phasenverschiebungen können z.B. chaotisch und/oder stochastisch variiert werden. Ferner können die visuellen Stimulationssignale unterschiedliche Polaritäten aufweisen. Im Falle eines Sinussignals als Stimulationssignal kann beispielsweise das Sinussignal von zwei Segmenten zeitgleich, aber mit umgekehrter Polarität appliziert werden (entspricht einer Phasenverschiebung von 180°).

Des Weiteren ist es möglich, dass jedes der Segmente 41 bis 44 ein Sinussignal mit jeweils unterschiedlicher Frequenz appliziert. Beispielsweise kann eines der Segmente ein Sinussignal mit 5 Hz und die anderen drei Segmente können Sinussignale mit 4 Hz, 3 Hz bzw. 2 Hz applizieren (d.h. im Falle einer Transmissionsbrille ändert sich die Transmission des jeweiligen Segments 41 bis 44 mit der entsprechenden Frequenz). Anstelle von Sinussignalen können auch andere (oszillierende) Signalformen, z.B. Rechtecksignale, mit der entsprechenden Grundfrequenz verwendet werden. Die Signale brauchen nicht zeitversetzt appliziert werden, sondern die Segmente 41 bis 44 können die Stimulationssignale beispielsweise auch gleichzeitig erzeugen. Die Stimulationssignale können kontinuierlich über einen längeren Zeitraum hinweg appliziert werden, es können aber auch Pausen während der Applikation eingehalten werden.

Die Applikation von visuellen Stimulationssignalen mit unterschiedlichen Frequenzen führt nicht notwendigerweise zu einem raschen Zurücksetzen der Phase der neuronalen Aktivität in den jeweiligen stimulierten Subpopulationen, jedoch wird durch die Stimulation mit diesen Signalen den jeweils stimulierten Subpopulationen über einen gewissen Zeitraum hinweg eine bestimmte, von der jeweiligen Stimulationsfrequenz abhängige Phase aufgezwungen. Letztlich führt auch dies zu einer Desynchronisation der gesamten Neuronenpopulation.

Alle vorstehend beschriebenen Stimulationsformen können auch in einem "closed loop"-Modus durchgeführt werden. Außerdem ist es denkbar, dass die Stimulation durch den Patienten gestartet wird, beispielsweise durch eine telemetrische Aktivierung. In diesem Fall kann der Patient die Stimulation für einen vorgegebenen Zeitraum von z.B. 5 Minuten aktivieren oder der Patient kann die Stimulation selbsttätig starten und beenden.

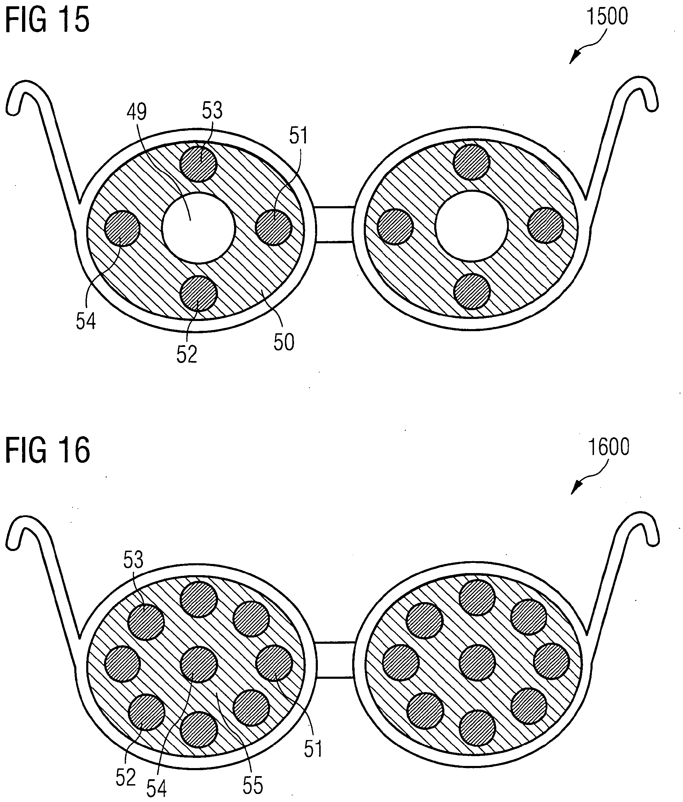

In Fig. 15 ist als weitere Ausführungsform der Stimulationseinheit 11 eine partiell durchsichtige Lichtbrille 1500 schematisch dargestellt. Bei der partiell durchsichtigen Lichtbrille 1500 wird kein Glas verwendet, dessen Transmission variiert werden kann. Vielmehr ist nur ein Teil 49 jedes der Brillengläser durchsichtig, während der übrige Teil 50 der Brillengläser undurchsichtig ist. An mindestens einem Ort ist pro Brillenglas eine Lichtquelle angeordnet. Die Lichtquelle kann z.B. eine Leuchtdiode oder ein Glasfaserkabel sein, das z.B. das Licht einer an anderer Stelle befestigten Leuchtdiode oder eines anderen Leuchtmittels zu dieser Stelle am Brillenglas weiterleitet. Die in Fig. 15 gezeigte Lichtbrille 1500 verfügt pro Brillenglas über vier Lichtquellen 51, 52, 53 und 54. Die Lichtbrille 1500 kann aber auch über jede andere Anzahl von Lichtquellen verfügen, die in einer beliebigen Geometrie angeordnet sein können. Ferner kann auch der durchsichtige Teil 49 anders ausgestaltet sein als in Fig. 15 dargestellt.

Der Patient kann nur durch den durchsichtigen Teil 49 der Brillengläser schauen. Wenn dieser Teil im Vergleich zum gesamten Brillenglas klein ist, wird der Patient gezwungen, seine Augen relativ zur Brille konstant positioniert zu halten. Die Lichtquellen 51 bis 54 reizen nur die Netzhaut des Patienten, während sie einen Betrachter auf der anderen Seite der Brille nicht visuell stimulieren. Die unterschiedlichen Lichtquellen 51 bis 54 reizen beispielsweise bestimmte Teilgebiete der Retina des Patienten. Der Zwischenraum zwischen Brillenrand und Gesicht kann lichtdicht abgeschlossen sein (nicht dargestellt).

In Fig. 16 ist als weitere Ausführungsform der Stimulationseinheit 11 eine undurchsichtige Lichtbrille 1600 schematisch dargestellt. Bei der undurchsichtigen Lichtbrille 1600 ist das Brillenglas 55 vollständig undurchsichtig. An mindestens einem Ort jedes der Brillengläser 55 ist eine Lichtquelle angebracht. Die Lichtquellen können genauso wie bei der partiell durchsichtigen Lichtbrille 1500 ausgestaltet sein, also z.B. als Leuchtdioden oder Glasfaserkabel. Bei dem in Fig. 16 gezeigten Beispiel weist jedes der Brillengläser neun Lichtquellen auf. Vier dieser Lichtquellen sind mit den Bezugszeichen 51 bis 54 versehen. Die Lichtbrille 1600 kann aber auch jede andere Anzahl von Lichtquellen aufweisen, die in einer beliebigen Art angeordnet sein können.

Der Patient kann nicht durch die Brillengläser schauen, sondern er wird ausschließlich durch die Lichtquellen visuell gereizt. Die Lichtquellen reizen - wie bei der partiell durchlässigen Lichtbrille 1500 - nur die Netzhaut des Patienten. Die unterschiedlichen Lichtquellen reizen bestimmte Teilgebiete der Retina des Patienten. Der Zwischenraum zwischen Brillenrand und Gesicht kann lichtdicht abgeschlossen sein (nicht dargestellt).

Die undurchsichtige Lichtbrille 1600 kann ein Fixation-Target enthalten, welches der Patient angenehm (z.B. ohne Blendungseffekte) fixieren kann. Durch die Instruktion, während der Therapie das Fixation-Target zu fixieren, wird verhindert, dass der Patient mit Augenfolgebewegungen den unterschiedlichen, aufleuchtenden Lichtquellen folgt. In letzterem Falle würde vor allem der zentrale Teil der Retina, die Fovea, gereizt, während mit einem Fixation-Target die unterschiedlichen Teile der Retina gereizt werden können.

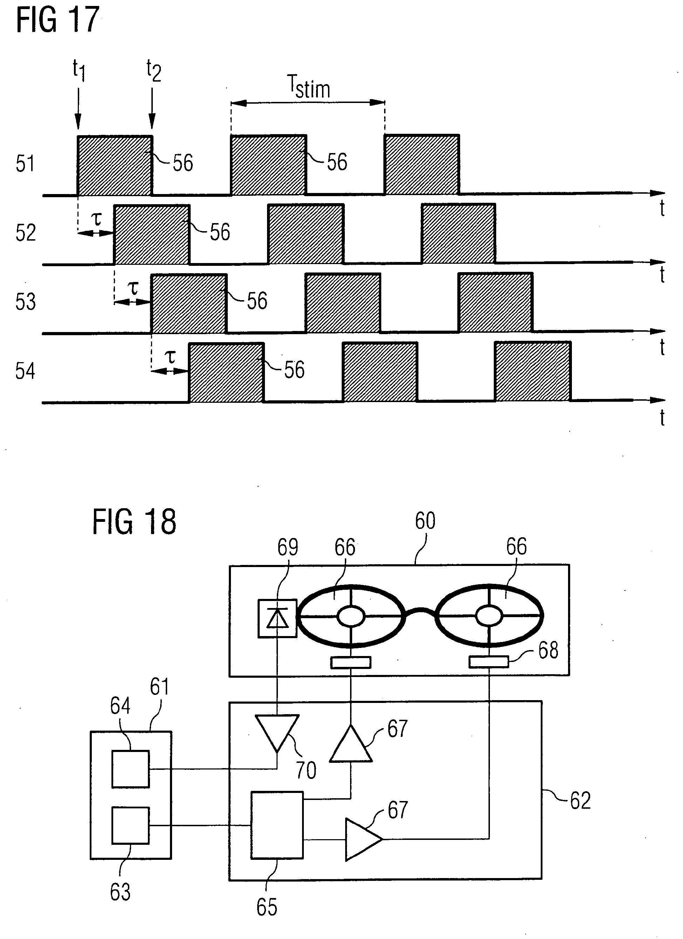

Ein Stimulationsverfahren, das beispielsweise mit den Lichtbrillen 1500 oder 1600 durchgeführt werden kann, ist in Fig. 17 schematisch dargestellt. In Fig. 17 sind untereinander die von den Lichtquellen 51 bis 54 der Lichtbrille 1500 oder 1600 applizierten visuellen Stimulationssignale 56 gegen die Zeit t aufgetragen.

Das in Fig. 17 dargestellte Verfahren entspricht im Wesentlichen dem in Fig. 11 gezeigten Verfahren für die Transmissionsbrille 1000. Bei dem in Fig. 17 dargestellten Verfahren appliziert jede der Lichtquellen 51 bis 54 periodisch das visuelle Stimulationssignal 56. Die Frequenz $f_{stim} = 1/T_{stim}$, mit welcher die visuellen Stimulationssignale 56 pro Lichtquelle 51 bis 54 wiederholt werden, kann im Bereich von 1 bis 30 Hz und insbesondere im Bereich von 5 bis 20 Hz liegen, kann aber auch kleinere oder größere Werte annehmen.

Zur einfacheren Darstellung ist in Fig. 17 das Stimulationsverfahren nur für vier Lichtquellen 51 bis 54 dargestellt. Dieses Verfahren kann jedoch in entsprechender Weise auf eine beliebige Anzahl von Lichtquellen erweitert werden.

Bei der Erzeugung der visuellen Stimulationssignale 56 mittels Lichtquellen wird typischerweise die betreffende Lichtquelle zum Zeitpunkt $t_1$ eingeschaltet und zum Zeitpunkt $t_2$ ausgeschaltet. Die maximale Amplitude (Helligkeit) der einzelnen Lichtreize liegt beispielsweise in einem Bereich von 1 bis 20 cd/m$^2$. Während der Stimulation, d.h. während der Zeitspanne zwischen $t_1$ und $t_2$, können auch kleinere Helligkeitswerte verwendet werden.

Alle im Zusammenhang mit den Fig. 11 bis 14 beschriebenen Ausgestaltungen können in entsprechender Weise auch auf die Stimulation mittels der Lichtbrillen 1500 bzw. 1600 übertragen werden.

Als weitere Alternative zu den Transmissionsbrillen 900 und 1000 sowie den Lichtbrillen 1500 und 1600 kann beispielsweise auch eine Videobrille zur Applikation der visuellen Stimulationssignale verwendet werden. Bei dieser Stimulationsart werden ein Videobild oder ein Videofilm in das Gesichtsfeld des Patienten projiziert. Das Videobild bzw. der Videofilm können in Segmente unterteilt sein, und die Helligkeit der einzelnen Segmente kann analog zu den oben beschriebenen Stimulationsverfahren variiert werden. Das Videobild bzw. der Videofilm können entweder vorproduziert werden oder es können ein oder mehrere Videokameras an der Brille angebracht sein, über die das Videobild bzw. der Videofilm aufgenommen werden.

In Fig. 18 ist als weiteres Ausführungsbeispiel eine segmentierte Transmissionsbrille 60 mit einer Ansteuerungselektronik schematisch dargestellt. Die Transmissionsgläser sind vorliegend mittels LCD (Liquid Crystal Display)-Technologie realisiert worden. Die Ansteuerungselektronik besteht aus einem Vorschaltmodul 61 und einem Ansteuerungsmodul 62. Das Vorschaltmodul 61 weist einen Eingang 63 und einen Ausgang 64 auf, über welche die Transmissionsbrille 60 mit der Steuereinheit 10 verbunden werden kann. Ein in den Eingang 63 eingespeistes Eingangssignal wird an eine Einstelleinheit 65 weitergeleitet, welche die Transmission der einzelnen Segmente 66 der Transmissionsbrille 60 einstellt. Die Pegel der von der Einstelleinheit 65 erzeugten Steuersignale werden zuvor noch von Verstärkereinheiten 67 auf geeignete Werte verstärkt. Die Transmission jedes Segments 66 der Transmissionsbrille 60 kann individuell gesteuert werden. Dazu ist jedes der Segmente 66 mit einem Anschluss 68 versehen, über den das jeweilige Steuersignal eingespeist werden kann.

Die Transmissionsbrille 60 kann des Weiteren über eine Photodiode 69 verfügen, die beispielsweise in ein Brillenglas integriert sein kann. Mittels der Photodiode 69 kann die Amplitude (Helligkeit) des Umgebungslichts detektiert werden, um anhand dieses Wertes die maximale Modulationsamplitude, also den Kontrast der Transmissionsbrille 60 einstellen zu können. Das von der Photodiode 69 generierte Signal wird von einer Verstärkereinheit 70 vorverstärkt und am Ausgang 64 bereitgestellt.

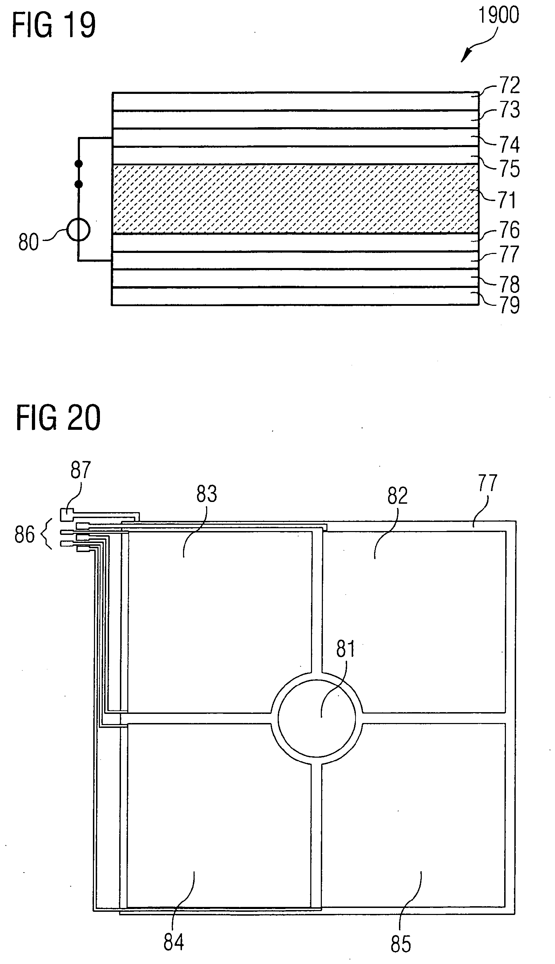

Fig. 19 zeigt schematisch einen Querschnitt durch ein LCD-Transmissionsglas 1900. Das LCD-Transmissionsglas 1900 besteht aus einem TN (Twisted Nematic)-Flüssigkristall 71, der in einen Stapel aus aktiven und passiven Schichten eingefügt ist. Wie in Fig. 19 dargestellt ist, sind auf der einen Seite des TN-Flüssigkristalls 71 eine durchsichtige Vorderseitenglasplatte 72, ein Polarisator 73, eine segmentierte, durchsichtige und elektrisch leitfähige Schicht 74 sowie eine Ausrichtungsschicht 75 angeordnet. Auf der anderen Seite des TN-Flüssigkristalls 71 sind eine Ausrichtungsschicht 76, eine durchgehende, durchsichtige und elektrisch leitfähige Schicht 77, ein Polarisator 78 und eine durchsichtige Rückseitenglasplatte 79 angeordnet. Der Abstand zwischen den beiden elektrisch leitfähigen Schichten 74 und 77 beträgt typischerweise zwischen 2 und 20 $\mu$m. Um geeignete Abstände zwischen den einzelnen Schichten einzuhalten, können Abstandshalter eingesetzt werden, die z.B. aus kleinen Glaskugeln oder -stäben bestehen.

Zwischen den beiden elektrisch leitfähigen Schichten 74 und 77 kann mit Hilfe einer Spannungsquelle 80 eine Spannung angelegt werden, mittels derer sich die Transmission des auf das LCD-Transmissionsglas 1900 einfallenden Umgebungslichts einstellen lässt. Die Polarisationsrichtungen der Polarisatoren 73 und 78 können senkrecht zueinander ausgerichtet sein. Das unpolarisierte Umgebungslicht wird durch den Polarisator 73 linear polarisiert. Durch den TN-Flüssigkristall 71 erfährt das linear polarisierte Licht eine 90°-Drehung, sodass die Polarisationsrichtung des Lichts danach der Polarisationsrichtung des Polarisators 78 entspricht und diesen ohne Intensitätsverlust durchläuft. Das mittels der Spannungsquelle 80 erzeugte elektrische Feld verändert die durch den TN-Flüssigkristall 71 bewirkte Drehung der Polarisation, was eine Veränderung der optischen Transmission zur Folge hat.

Das vorstehend beschriebene Prinzip zur Variation der Transmission lässt sich auf verschiedene Arten modifizieren. Beispielsweise können zwei "Guest-Host"-Flüssigkristallzellen senkrecht zueinander ausgerichtet werden, wodurch beide Richtungen des polarisierten Lichts im ausgeschalteten Zustand absorbiert werden und hohe Kontrastverhältnisse ohne Polarisatoren ermöglicht werden. Der durchsichtige Zustand wird erreicht, indem eine geeignete Spannung angelegt wird, welche die Polarisationsrichtungen beider Flüssigkristallzellen gleich ausrichtet.

Für den Flüssigkristall können neben dem oben genannten TN-Flüssigkristall auch andere Arten von Flüssigkristallen verwendet werden, beispielsweise eignen sich sogenannte "Polymer Stabilized Cholestric Textured"- oder "Electrically Commanded Surface"-Flüssigkristalle.

Zur Herstellung der elektrisch leitfähigen Schichten 74 und 77 kann beispielsweise Indiumzinnoxid (ITO; indium tin oxide) verwendet werden. Indiumzinnoxid kann z.B. mittels Elektronenstrahlverdampfung, physikalischer Gasphasenabscheidung oder Sputtern auf den TN-Flüssigkristall 71 aufgebracht werden.

Damit die einzelnen Segmente der Transmissionsbrille separat angesteuert werden können, ist eine der beiden elektrisch leitfähige Schichten 74 und 77 segmentiert. In Fig. 20 ist eine Segmentierung der elektrisch leitfähigen Schicht 74 beispielhaft dargestellt. Die elektrisch leitfähige Schicht 74 besteht vorliegend aus fünf Segmenten 81 bis 85, die elektrisch voneinander isoliert sind. Jedem der Segmente 81 bis 85 ist ein eigener Anschluss 86 zugeordnet, über den die einzelnen Segmente 81 bis 86 jeweils separat angesteuert werden können. Die elektrisch leitfähige Schicht 77 weist ebenfalls einen eigenen Anschluss 87 auf. Zur Ansteuerung eines der Segmente 81 bis 85 wird eine geeignete Spannung zwischen den jeweiligen Anschluss 86 und den Anschluss 87 angelegt.

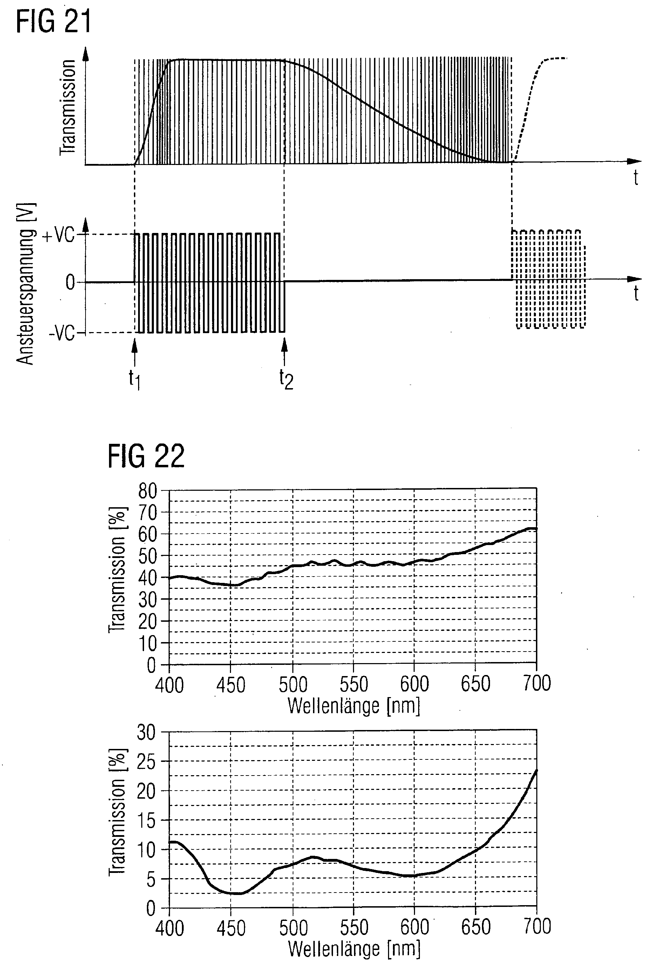

In Fig. 21 ist die Ansteuerung eines der Segmente 81 bis 85 und das Transmissionsverhalten des entsprechenden Segments als Antwort auf die angelegte Ansteuerspannung dargestellt. Wie aus Fig. 21 ersichtlich, wird zum Zeitpunkt $t_1$ begonnen, die Transmission auf den maximalen Wert hochzufahren und zum Zeitpunkt $t_2$ wird die Transmission wieder auf den minimalen Wert heruntergefahren. Im angeschalteten Zustand, d.h. zwischen den Zeitpunkten $t_1$ und $t_2$ wird keine Gleichspannung an die elektrisch leitfähigen Schichten 74 und 77 angelegt, vielmehr wird die Ansteuerspannung zwischen den Spannungswerten +VC und -VC mit einer Frequenz im Bereich von 10 Hz bis 5 kHz hin- und hergeschaltet. Der zeitliche Mittelwert der angelegten Spannung beträgt dabei 0 V. Das Anlegen der amplitudenmodulierten Spannung verhindert eine Migration von Ionen, die nicht-reversibel die LC-Zelle zerstören würde. Um das Eindringen von Ionen in den TN-Flüssigkristall 71 zu unterbinden, können die elektrisch leitfähigen Schichten 74 und 77 ferner mit Schutzschichten versehen werden, die z.B. aus Siliziumdioxid bestehen können.

Mittels der Spannung +VC/-VC wird der maximale Kontrast der Segmente 81 bis 85 generiert. Durch eine Modulation der an die elektrisch leitfähigen Schichten 74 und 77 angelegten Spannung (V < VC) kann die Transmission/Extinktion angepasst werden und variable Graustufen können erzeugt werden.

In Fig. 22 ist beispielhaft das Transmissionsspektrum von zwei senkrecht zueinander ausgerichteten "Guest-Host"-Flüssigkristallzellen dargestellt. Die Dicke der Flüssigkristallschicht beträgt in dem vorliegenden Beispiel 4 μm. Die obere Abbildung zeigt die "Guest-Host"-Flüssigkristallzellen im angeschalteten Zustand mit maximaler Transmission und die untere Abbildung im ausgeschalteten Zustand mit minimaler Transmission.

Die in den Fig. 18 bis 22 gezeigten Ausgestaltungen einer Transmissionsbrille sind lediglich beispielhaft zu verstehen. Eine Transmissionsbrille zur Realisierung der in Fig. 1 gezeigten Stimulationseinheit 11 kann auch andere Ausgestaltungsmerkmale aufweisen.

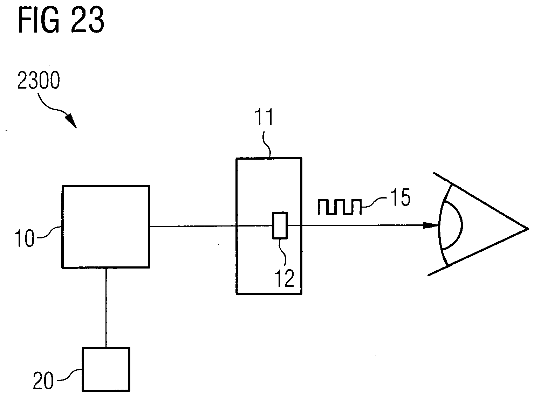

In Fig. 23 ist schematisch eine Vorrichtung 2300 dargestellt, die in weiten Teilen der aus Fig. 3 bekannten Vorrichtung 300 entspricht und deren baugleiche oder zumindest bauähnliche Komponenten daher mit denselben Bezugszeichen gekennzeichnet sind. Die Vorrichtung 2300 enthält wie die Vorrichtung 300 eine Stimulationseinheit 11, die im Unterschied zur Vorrichtung 300 aber nicht notwendigerweise mindestens zwei Stimulationselemente enthalten muss. Die Vorrichtung 2300 kann beispielsweise nur ein einziges Stimulationselement 12 zur Erzeugung von visuellen Stimulationssignalen 15 aufweisen, es kann jedoch auch eine beliebige, größere Anzahl von Stimulationselementen vorgesehen sein. Genauso wie die Vorrichtung 300 umfasst die Vorrichtung 2300 eine Messeinheit 20, welche am Patienten aufgenommene Messsignale bereitstellt und diese an eine Steuereinheit 10 weiterleitet. Die Steuereinheit 10 verwendet die Messsignale, um daraus Steuersignale zu generieren, mit denen das bzw. die Stimulationselemente 12 angesteuert werden.

Bei der Messeinheit 20 kann es sich um nicht-invasive Sensoren handeln, wie z.B. Elektroenzephalographie (EEG)-Elektroden, Magnetenzephalographie (MEG)-Sensoren, Akzelerometer, Elektromyographie (EMG)-Elektroden und Sensoren zur Bestimmung von Blutdruck, Atmung oder Hautleitwiderstand. Ferner kann die Messeinheit 20 in Form eines oder mehrerer Sensoren in den Körper des Patienten implantiert sein. Als invasive Sensoren können beispielsweise epikortikale, intrakortikale oder subkutane Elektroden dienen. Insbesondere kann mittels der Messeinheit 20 die physiologische Aktivität in dem stimulierten Zielgebiet oder einem damit verbundenen Gebiet gemessen werden.

Die Vorrichtung 2300 kann zur Desynchronisation einer Neuronenpopulation mit einer krankhaft synchronen und oszillatorischen neuronalen Aktivität eingesetzt werden. Die Vorrichtung 2300 kann die gleichen Ausgestaltungen wie die Vorrichtung 300 aufweisen.

Die von der Messeinheit 20 aufgenommenen Messsignale können direkt oder gegebenenfalls nach einem oder mehreren Verarbeitungsschritten in visuelle Stimulationssignale 15 umgesetzt werden und von der Stimulationseinheit 11 appliziert werden. Beispielsweise können die Messsignale verstärkt und gegebenenfalls nach mathematischer Verrechnung (z.B. nach Mischung der Messsignale) mit einer Zeitverzögerung und linearen und/oder nicht-linearen Verrechnungsschritten als Steuersignale für die Stimulationseinheit 11 verwendet werden. Der Verrechnungsmodus kann hierbei so gewählt werden, dass der krankhaften neuronalen Aktivität entgegengewirkt wird und das Stimulationssignal mit abnehmender krankhafter neuronaler Aktivität ebenfalls verschwindet oder zumindest deutlich in seiner Stärke reduziert wird.

Im Folgenden werden lineare und nicht-lineare Verarbeitungsschritte beschrieben, mit denen die mit Hilfe der Messeinheit 20 gewonnenen Messsignale prozessiert werden können, bevor sie in den oder die Steuereingänge des oder der Stimulationselemente 12 eingespeist werden. Sowohl die linearen als auch die nicht-linearen Verarbeitungsschritte können in gleicher Weise sowohl in der Vorrichtung 300 als auch in der Vorrichtung 2300 eingesetzt werden. Bei einer nicht-linearen Verarbeitung der Messsignale wird nicht die Phase der neuronalen Aktivität in den jeweiligen stimulierten Subpopulationen zurückgesetzt, sondern die Synchronisation in der krankhaft aktiven Neuronenpopulation wird unterdrückt, indem der Sättigungsprozess der Synchronisation beeinflusst wird.

Bei einer linearen Verarbeitung eines von der Messeinheit 20 gewonnenen Messsignals kann das Messsignal beispielsweise gefiltert und/oder verstärkt und/oder mit einer Zeitverzögerung beaufschlagt werden, bevor mit dem so verarbeiteten Signal die Stimulationseinheit 11 bzw. die in der Stimulationseinheit 11 angeordneten Stimulationselemente 12 (und 13) angesteuert werden. Als Beispiel sei angenommen, dass das Messsignal mittels einer epikortikalen oder intrakortikalen Elektrode aufgenommen worden sei und die pathologische Aktivität im Zielgebiet wiedergebe. Dementsprechend ist das Messsignal eine Sinusschwingung mit einer Frequenz im Bereich von 1 bis 30 Hz. Weiterhin sei beispielhaft angenommen, dass das Messsignal eine Frequenz von 5 Hz aufweise. Das Messsignal kann mittels eines Bandpassfilters mit einem Durchlassbereich im Bereich von 5 Hz gefiltert werden und mittels eines Verstärkers so verstärkt werden, dass es für die Ansteuerung der Stimulationseinheit 11 geeignete Pegel aufweist. Anschließend wird die so erhaltene verstärkte Sinusschwingung zur Ansteuerung der Stimulationseinheit 11 verwendet. Übertragen auf das in Fig. 11 gezeigte Stimulationsverfahren bedeutet dies, dass die rechteckförmigen Stimulationssignale 45 in diesem Fall durch eine Sinusschwingung mit einer Frequenz von 5 Hz ersetzt werden.

Sofern zur Stimulation eine Mehrzahl von Stimulationselementen herangezogen wird, kann das Messsignal mit den in Fig. 11 gezeigten Verzögerungen $\tau$ beaufschlagt werden, bevor es als Steuersignal in die entsprechenden Stimulationselemente eingespeist wird.

Im Folgenden wird anhand eines Beispiels erläutert, wie ein von der Messeinheit 20 gewonnenes Messsignal einer nichtlinearen Prozessierung unterworfen werden kann, bevor es als Ansteuerungssignal der Stimulationseinheit 11 verwendet wird. Genauso wie bei der linearen Verarbeitung kann das Messsignal auch hier gefiltert und/oder verstärkt und/oder mit einer Zeitverzögerung beaufschlagt werden.

Ausgangspunkt ist eine Gleichung für das Ansteuerungssignal S (t) :

$$S(t) = K \cdot \overline{Z}^2(t) \cdot \overline{Z}^*(t - \tau) \qquad (1)$$

In Gleichung (1) sind K ein Verstärkungsfaktor, der geeignet gewählt werden kann, und $\overline{z}(t)$ eine mittlere Zustandsvariable des Messsignals. $\overline{z}(t)$ ist eine komplexe Variable und kann folgendermaßen dargestellt werden:

$$\overline{Z}(t) = X(t) + iY(t), \qquad (2)$$

wobei X(t) z.B. dem neurologischen Messsignal entsprechen kann. Da die betrachteten Frequenzen im Bereich von 10 Hz = 1/100ms = $1/T_\alpha$ liegen, kann der Imaginärteil Y(t) durch $X(t - \tau_\alpha)$ angenähert werden, wobei beispielsweise $\tau_\alpha = T_\alpha/4$ gilt. Damit ergibt sich:

$$S(t) = K \cdot [X(t) + iX(t - \tau_\alpha)]^2 \cdot [X(t - \tau) - iX(t - \tau - \tau_\alpha)] \qquad (3)$$

Gleichung (3) kann folgendermaßen umgeformt werden:

$$\begin{aligned} S(t) = K \cdot [&X(t)^2 \cdot X(t - \tau) + i2X(t) \cdot X(t - \tau_\alpha) \cdot X(t - \tau) - X(t - \tau_\alpha) \cdot X(t - \tau) \\ &- iX(t - \tau - \tau_\alpha) \cdot X(t)^2 + 2X(t) \cdot X(t - \tau_\alpha) \cdot X(t - \tau - \tau_\alpha) \\ &+ iX(t - \tau - \tau_\alpha) \cdot X(t - \tau_\alpha)] \end{aligned} \qquad (4)$$

Als Ansteuerungssignal für die Stimulationseinheit 11 wird der Realteil aus Gleichung (4) verwendet:

$$real[S(t)] = K \cdot [X(t)^2 \cdot X(t - \tau) - X(t - \tau_\alpha) \cdot X(t - \tau) + 2X(t) \cdot X(t - \tau_\alpha) \cdot X(t - \tau - \tau_\alpha)] \qquad (5)$$

**Patentansprüche**

1. Vorrichtung (100; 300) umfassend:

   - eine Steuereinheit (10), und
   - eine Stimulationseinheit (11) mit einer Mehrzahl von Stimulationselementen (12, 13), die visuelle Stimulationssignale erzeugen,
   - wobei N der Stimulationselemente (12, 13) die visuellen Stimulationssignale an unterschiedlichen räumlichen Orten erzeugen, wobei N ≥ 2 gilt, und
   - wobei die N Stimulationselemente (12, 13) die visuellen Stimulationssignale an vorgegebenen Orten im Gesichtsfeld des Patienten erzeugen,
   - wobei die Stimulationselemente (12, 13) derart ausgestaltet sind, dass sie visuelle Stimulationssignale erzeugen, welche bei einer Aufnahme über ein Auge eines Patienten und Weiterleitung an Neuronen, die eine krankhaft synchrone und oszillatorische neuronale Aktivität aufweisen, die Phase der neuronalen Aktivität der Neuronen zurücksetzen,
   - die N Stimulationselemente (12, 13) derart im Gesichtsfeld des Patienten angeordnet sind, dass die von den N Stimulationselementen (12, 13) erzeugten visuellen Stimulationssignale unterschiedliche Subpopulationen einer Neuronenpopulation stimulieren, die eine krankhaft synchrone und oszillatorische neuronale Aktivität aufweist,

- die Steuereinheit (10) die Stimulationseinheit (11) derart ansteuert, dass die N Stimulationselemente (12, 13) die visuellen Stimulationssignale zeitversetzt zueinander erzeugen, so dass die Phasen der neuronalen Aktivität der Subpopulationen zu unterschiedlichen Zeitpunkten zurückgesetzt werden,
- die N Stimulationselemente (12, 13) derart ausgebildet sind, dass der zeitliche Versatz zwischen jeweils zwei aufeinander folgenden, von unterschiedlichen der N Stimulationselemente (12, 13) erzeugten visuellen Stimulationssignalen $1/ (f_{stim} \times N)$ beträgt und $f_{stim}$ eine Frequenz im Bereich von 1 bis 30 Hz ist, und
- **dadurch gekennzeichnet, dass** die Steuereinheit (10) derart ausgebildet ist, dass die Dauer der von den N Stimulationselementen (12, 13) erzeugten visuellen Stimulationssignale einstellbar ist.

2. Vorrichtung (100; 300) nach Anspruch 1, wobei die Stimulationseinheit (11) für eine Dauer von $M/f_{stim}$ die visuelle Stimulationssignale erzeugt und anschließend für eine Dauer von $L/f_{stim}$ keine visuellen Stimulationssignale erzeugt, wobei M und L ganze Zahlen sind.

3. Vorrichtung (100; 300) nach Anspruch 1 oder 2, wobei die Stimulationseinheit eine Transmissionsbrille (1000) ist und die Transmissionsbrille (1000) eine Mehrzahl von Segmenten (41, 42, 43, 44) aufweist und die Transmissionseigenschaften der Segmente (41, 42, 43, 44) von der Steuereinheit (10) gesteuert werden.

4. Vorrichtung (100; 300) nach Anspruch 1 oder 2, wobei die Stimulationseinheit eine Brille (1500; 1600) ist und die Stimulationselemente (12, 13) in Form einer Mehrzahl von Lichtquellen (51, 52, 53, 54) ausgebildet sind.

5. Vorrichtung (100; 300) nach Anspruch 4, wobei die Brille (1600) undurchsichtig ist.

6. Vorrichtung (300) nach einem der vorhergehenden Ansprüche, wobei die Vorrichtung (300) eine Messeinheit (20) zum Aufnehmen von am Patienten gemessenen Messsignalen umfasst.

7. Vorrichtung (300) nach Anspruch 6, wobei die Steuereinheit (10) die Stimulationselemente (12, 13) anhand der Messsignale derart ansteuert, dass die Stimulationselemente (12, 13) die Messsignale in visuelle Stimulationssignale umsetzen.

## Claims

1. Device (100; 300) comprising:

   - a control unit (10), and
   - a stimulation unit (11) having a plurality of stimulation elements (12, 13) which generate visual stimulation signals
   - wherein N of the stimulation elements (12, 13) generate the visual stimulation signals at different spatial locations, wherein $N \geq 2$ holds, and
   - wherein the N stimulation elements (12, 13) generate the visual stimulation signals at predetermined locations in the field of view of the patient,
   - wherein the simulation elements (12, 13) are configured in such a way that they generate visual stimulation signals which, when recorded via an eye of a patient and transmitted to neurons having a morbidly synchronous and oscillatory neuronal activity, reset the phase of the neuronal activity of the neurons,
   - the N stimulation elements (12, 13) are arranged in the field of view of the patient in such a way that the visual simulation signals generated by the N stimulation elements (12, 13) stimulate different subpopulations of a neuron population having a morbidly synchronous and oscillatory neuronal activity,
   - the control unit (10) controls the stimulation unit (11) in such a way that the N stimulation elements (12, 13) generate the visual stimulation signals with a time shift between each other so that the phases of the neuronal activity of the subpopulations are reset at different points in time,
   - the N stimulation elements (12, 13) are configured in such a way that the time shift between each of two consecutive visual stimulation signals generated by different ones of the N stimulation elements (12, 13) is $1 / (f_{stim} \times N)$ and $f_{stim}$ is a frequency in the range of 1 to 30 Hz, and

   **characterized in that**

   - the control unit (10) is configured in such a way that the duration of the visual stimulation signals generated by the N stimulation elements (12, 13) is adjustable.

**2.** Device (100; 300) according to claim 1, wherein the stimulation unit (11) generates the visual stimulation signals for a duration of M / $f_{stim}$ and subsequently for a duration of L / $f_{stim}$ generates no visual stimulation signals, with M and L being integers.

**3.** Device (100; 300) according to claim 1 or 2, wherein the stimulation unit is a pair of transmission goggles (1000) and the pair of transmission goggles (1000) has a plurality of segments (41, 42, 43, 44) and the transmission properties of the segments (41, 42, 43, 44) are controlled by the control unit (10).

**4.** Device (100; 300) according to claim 1 or 2, wherein the stimulation unit is a pair of goggles (1500; 1600) and the stimulation elements (12, 13) are configured in form of a plurality of light sources (51, 52, 53, 54).

**5.** Device (100; 300) according to claim 4, wherein the pair of goggles (1600) is opaque.

**6.** Device (300) according to any one of the preceding claims, wherein the device (300) comprises a measuring unit (20) for recording measurement signals measured on the patient.

**7.** Device (300) according to claim 6, wherein the control unit (10) controls the stimulation elements (12, 13) on the basis of the measurement signals in such a way that the stimulation elements (12, 13) convert the measurement signals into visual stimulation signals.

**Revendications**

**1.** Dispositif (100 ; 300) comprenant :

- un module de commande (10), et
- un module de stimulation (11) avec une pluralité d'éléments de stimulation (12, 13) qui génèrent des signaux de stimulation visuelle,
- dans lequel N des éléments de stimulation (12, 13) génèrent les signaux de stimulation visuelle à différents lieux spatiaux, dans lequel N ≥ 2 est valable, et
- dans lequel les N éléments de stimulation (12, 13) génèrent les signaux de stimulation visuelle à des lieux prédéfinis dans le champ visuel du patient,
- dans lequel les éléments de stimulation (12, 13) sont configurés de telle sorte qu'ils génèrent des signaux de stimulation visuelle qui reculent lors d'un enregistrement par le biais d'un œil d'un patient et d'une transmission à des neurones qui présentent une activité neuronale maladivement synchrone et oscillatoire la phase de l'activité neuronale des neurones,
- les N éléments de stimulation (12, 13) sont disposés dans le champ visuel du patient de telle sorte que les signaux de stimulation visuelle générés par les N éléments de stimulation (12, 13) stimulent différentes sous-populations d'une population de neurones qui présente une activité neuronale maladivement synchrone et oscillatoire,
- le module de commande (10) commande le module de stimulation (11) de telle sorte que les N éléments de stimulation (12, 13) génèrent les signaux de stimulation visuelle de manière décalée dans le temps les uns par rapport aux autres de sorte que les phases de l'activité neuronale des sous-populations sont reculées à différents moments,
- les N éléments de stimulation (12, 13) sont réalisés de telle sorte que le décalage dans le temps entre à chaque fois deux signaux de stimulation visuelle successifs, générés par différents éléments des N éléments de stimulation (12, 13) se monte à 1/($f_{stim}$ x N) et fstim est une fréquence dans la région de 1 à 30 Hz, et
- **caractérisé en ce que** le module de commande (10) est réalisé de telle sorte que la durée des signaux de stimulation visuelle générés par les N éléments de stimulation (12, 13) est réglable.

**2.** Dispositif (100 ; 300) selon la revendication 1, dans lequel le module de stimulation (11) génère les signaux de stimulation visuelle pendant une durée de M/$f_{stim}$, puis ne génère aucun signal de stimulation visuelle pendant une durée de L/$f_{stim}$, dans lequel M et L sont des nombres entiers.

**3.** Dispositif (100 ; 300) selon la revendication 1 ou 2, dans lequel le module de stimulation est une lunette de transmission (1000) et la lunette de transmission (1000) présente une pluralité de segments (41, 42, 43, 44) et les propriétés de transmission des segments (41, 42, 43, 44) sont commandées par le module de commande (10).

4.  Dispositif (100 ; 300) selon la revendication 1 ou 2, dans lequel le module de stimulation est une lunette (1500 ; 1600) et les éléments de stimulation (12, 13) sont réalisés sous forme d'une pluralité de sources lumineuses (51, 52, 53, 54).

5.  Dispositif (100 ; 300) selon la revendication 4, dans lequel la lunette (1600) est opaque.

6.  Dispositif (300) selon une des revendications précédentes, dans lequel le dispositif (300) comprend un module de mesure (20) pour l'enregistrement de signaux de mesure mesurés sur le patient.

7.  Dispositif (300) selon la revendication 6, dans lequel le module de commande (10) commande les éléments de stimulation (12, 13) à l'aide des signaux de mesure de telle sorte que les éléments de stimulation (12, 13) convertissent les signaux de mesure en signaux de stimulation visuelle.

FIG 1

100

11

12

15

16

10

13

14

FIG 2

y

17

18

φ18

19

12

13

φ19

x

FIG 3

11

300

12

15

16

10

13

14

20

FIG 4

FIG 5

FIG 6

**FIG 7**

**FIG 8**

FIG 9

900

21 22 40 23

FIG 10

1000

41 43 40 23

44

42

## FIG 11

## FIG 12

## FIG 13

## FIG 14

FIG 15

1500

FIG 16

1600

## FIG 17

## FIG 18

## FIG 19

1900

80

72
73
74
75
71
76
77
78
79

## FIG 20

87
86
83
82
77
81
84
85

## FIG 21

## FIG 22

# FIG 23

2300

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0164005 A2
- DE 19543405 A1
- DE 19905145 A1
- DE 10110004 A1

**In der Beschreibung aufgeführte Nicht-Patentliteratur**

- **B. A. WANDELL ; S. O. DUMOULIN ; A. A. BREWER.** Visual Field Maps in Human Cortex. *Neuron,* Oktober 2007, vol. 56, 366-383